# EUROPEAN PATENT APPLICATION

(11) **EP 4 006 005 A1**
(43) Date of publication of application: **01.06.2022**
(21) Application number: 20844251.7
(22) Date of filing: 22.07.2020
(51) Int. Cl.: C07C 4/10, C07C 9/15, C07C 11/22, C01B 3/00, C01B 3/26, C07B 61/00, B01J 37/08, B01J 37/34, C01B 32/184, C01B 32/194, B01J 21/18

(54) **ALKANE DEHYDROGENATION CATALYST, AND HYDROGEN PRODUCTION METHOD USING SAME**

(30) Priority: 24.07.2019 JP 2019135780
(71) Applicant: Osaka University, Suita-shi, Osaka 565-0871 (JP); Hosei University, Tokyo 102-8160 (JP); Daicel Corporation, Osaka 530-0011 (JP)
(72) Inventor: KUSAKABE, Koichi, Suita-shi, Osaka 565-0871 (JP); TAKAI, Kazuyuki, Tokyo 102-8160 (JP); NISHIKAWA, Masahiro, Tokyo 108-8230 (JP); LIU, Ming, Tokyo 108-8230 (JP)
(74) Representative: Grünecker Patent- und Rechtsanwälte PartG mbB
(86) International application number: PCT/JP2020/028503
(87) International publication number: WO 2021/015250

(57) **Abstract**

Provided are: a catalyst that is used in a reaction for producing hydrogen from an alkane without emitting CO₂; a method of producing hydrogen without emitting CO2 by using the catalyst; and a method of producing ammonia using, as a reducing agent, hydrogen produced using the catalyst. The alkane dehydrogenation catalyst according to the present disclosure contains a graphene having at least one type of structure selected from an atomic vacancy structure, a singly hydrogenated vacancy structure, a doubly hydrogenated vacancy structure, a triply hydrogenated vacancy structure, and a nitrogen-substituted vacancy structure. The graphene preferably has from 2 to 200 of the structure approximately per 100 nm² of the atomic film of the graphene. In addition, the hydrogen production method according to the present disclosure includes extracting hydrogen from an alkane by using the alkane dehydrogenation catalyst.

## Description

### Technical Field

The present disclosure relates to an alkane dehydrogenation catalyst and a hydrogen production method using the catalyst. The present disclosure claims priority from the Japanese patent application No. 2019-135780, filed in Japan on July 24, 2019, the contents of which are incorporated herein by reference.

### Background Art

Modern life has become increasingly dependent on electrical energy. However, CO₂ is emitted when generating electrical energy by burning fossil fuels, causing a greenhouse effect, which is problematic.

Therefore, as a renewable energy that does not emit CO₂, hydrogen has been attracting attention. When combined with oxygen, hydrogen can be used to generate electricity or be burned and used as thermal energy, during which CO₂ is not emitted.

It is known that hydrogen, which is useful in this manner, can be produced from fossil fuels by steam reforming (Patent Document 1). It is also known that hydrogen can be produced by a carbon monoxide shift reaction (Patent Document 2). However, with the methods described above, CO₂ is emitted when hydrogen is being produced.

### Citation List

### Patent Documents

Patent Document 1: JP 2014-185059 A
Patent Document 2: JP 2006-232610 A

### Summary of Invention

### Technical Problem

Therefore, an object of the present disclosure is to provide a catalyst that is used in a reaction for producing hydrogen from an alkane without emitting CO₂.

Another object of the present disclosure is to provide a method for producing hydrogen from an alkane without emitting CO₂ by using the catalyst.

### Solution to Problem

As a result of diligent research to solve the problems described above, the present inventors discovered that hydrogen can be extracted from an alkane without emitting CO₂ by using, as an alkane dehydrogenation catalyst, a graphene having at least one type of structure selected from: an atomic vacancy structure; a singly hydrogenated vacancy structure; a doubly hydrogenated vacancy structure; a triply hydrogenated vacancy structure; and a nitrogen-substituted vacancy structure. The present disclosure has been completed based on these findings.

That is, the present disclosure provides an alkane dehydrogenation catalyst containing a graphene having at least one type of structure selected from: an atomic vacancy structure; a singly hydrogenated vacancy structure; a doubly hydrogenated vacancy structure; a triply hydrogenated vacancy structure; and a nitrogen-substituted vacancy structure.

The present disclosure also provides the alkane dehydrogenation catalyst described above, wherein the graphene has from 2 to 200 of the at least one type of structure selected from: an atomic vacancy structure; a singly hydrogenated vacancy structure; a doubly hydrogenated vacancy structure; a triply hydrogenated vacancy structure; and a nitrogen-substituted vacancy structure, per 100 nm² of an atomic film of the graphene.

The present disclosure also provides a method of producing an alkane dehydrogenation catalyst, including colliding high-energy particles with a raw material graphene to obtain the alkane dehydrogenation catalyst.

The present disclosure also provides the method of producing an alkane dehydrogenation catalyst, wherein the raw material graphene is a graphene obtained by a detonation method.

The present disclosure also provides a method of producing hydrogen, including extracting hydrogen from an alkane by using the alkane dehydrogenation catalyst.

The present disclosure also provides the method of producing hydrogen, further including adsorbing-storing the hydrogen extracted from an alkane in an atomic vacancy site of the graphene.

The present disclosure also provides a hydrogen production apparatus producing hydrogen using the method.

The present disclosure also provides a method of producing ammonia, including producing hydrogen by the method, and reducing a nitrogen oxide using the produced hydrogen to obtain ammonia.

The present disclosure also provides an ammonia production apparatus producing ammonia using the method.

### Advantageous Effects of Invention

The alkane dehydrogenation catalyst according to an embodiment of the present disclosure enables extraction of hydrogen from an alkane without emitting CO₂ and without requiring significant energy. The extracted hydrogen can also be safely stored in the alkane dehydrogenation catalyst, and the stored hydrogen can be extracted as needed. Furthermore, a large amount of energy is not required when extracting hydrogen.

The hydrogen thus obtained is extremely useful as a renewable energy; even when the hydrogen is burned and used as thermal energy, CO₂ is not emitted.

Therefore, the hydrogen obtained by the method of producing hydrogen according to an embodiment of the present disclosure is a "carbon-free" energy that does not involve CO₂ emission during the entire process from production to use.

In addition, the hydrogen thus obtained can be used as an energy source for, for example, a fuel cell vehicle.

Furthermore, ammonia can be efficiently produced by using the hydrogen, obtained by the method of producing hydrogen, as a reducing agent for a nitrogen oxide. Here, ammonia is a substance having a high hydrogen density which liquefies under mild conditions. In addition, ammonia has the potential to be used as a fuel. In a case in which ammonia can be used as a fuel, fuel is not required when extracting energy from ammonia. Thus, ammonia is suitable for large-amount transportation and storage of hydrogen energy, and is extremely useful as an energy carrier.

Therefore, in a case in which the hydrogen, obtained by the method of producing hydrogen, is used to produce ammonia useful as described above, it is possible to safely store a large amount of hydrogen without emitting CO₂, and the hydrogen can be converted to energy as needed.

### Brief Description of Drawings

FIG. 1 is a schematic view illustrating an atomic vacancy structure of a V/graphene.
FIG. 2 is a schematic view illustrating a singly hydrogenated vacancy structure of a V₁/graphene.
FIG. 3 is a schematic view illustrating a doubly hydrogenated vacancy structure of a V₁₁/graphene.
FIG. 4 is a schematic view illustrating a triply hydrogenated vacancy structure of a V₁₁₁/graphene.
FIG. 5 is a schematic view illustrating a nitrogen-substituted vacancy structure of a V_{NCC}/graphene.
FIG. 6 is a schematic view illustrating a hydrogen adsorption-storage reaction and a hydrogen release reaction in an atomic vacancy site of a V₁/graphene.
FIG. 7 is a schematic view illustrating a hydrogen adsorption-storage reaction and a hydrogen release reaction in an atomic vacancy site of a V_{NCC}/graphene.

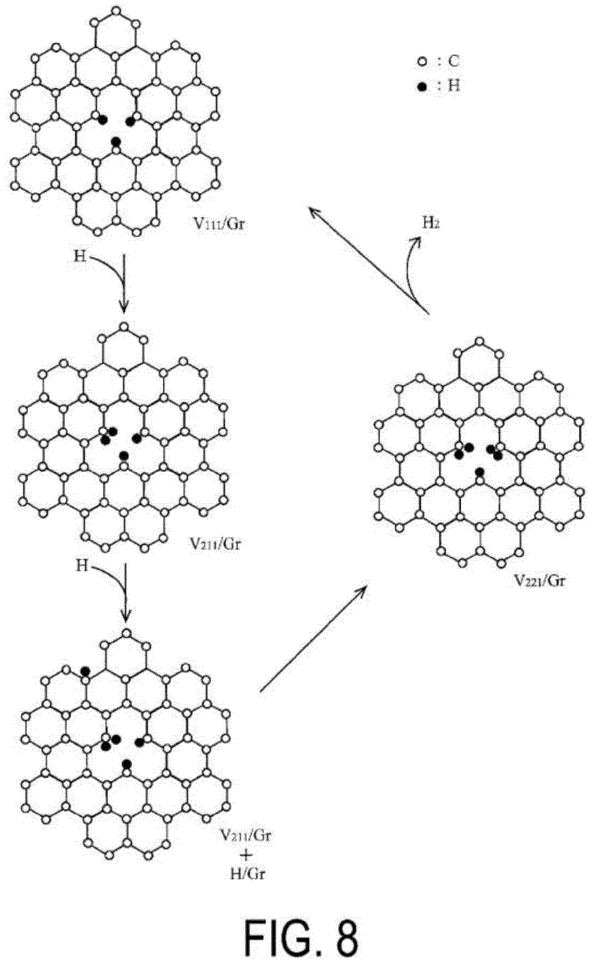

FIG. 8 is a schematic view illustrating a hydrogen adsorption-storage reaction and a hydrogen release reaction in an atomic vacancy site of a V₁₁₁/graphene.
FIG. 9 is a flow chart illustrating an example of a schematic configuration of a hydrogen production apparatus according to an embodiment of the present disclosure.
FIG. 10 is a schematic view illustrating a reaction vessel 11 having a mesh-like catalyst support structure.
FIG. 11 is a flow chart illustrating an example of a schematic configuration of an ammonia production apparatus according to an embodiment of the present disclosure.
FIG. 12 is a diagram illustrating a change of hydrogen amount in a catalyst (2) obtained in Example 2 before and after a reaction with n-butane.
FIG. 13 is a diagram illustrating changes of hydrogen amount at different sites of the catalyst (2) obtained in Example 2 before and after a reaction with an alkane.
FIG. 14 is a diagram illustrating a change of hydrogen amount in an atomic vacancy site of the catalyst (2) obtained in Example 2 before and after a reaction of an alkane.
FIG. 15 is a diagram illustrating evaluation results of a reaction path of and activation barrier between a V₁/graphene and n-octane determined by an electronic state calculation based on the Density Functional Theory.
FIG. 16 is a diagram illustrating evaluation results of a reaction path of and activation barrier between a V_{NCC}/graphene and n-octane determined by an electronic state calculation based on the Density Functional Theory.
FIG. 17 is a diagram illustrating multiple stages between FIG. 16 (1)-a and FIG. 16 (1)-b-2.
FIG. 18 is a diagram illustrating evaluation results of a reaction path of and activation barrier between a V₁₁₁/graphene and n-octane determined by an electronic state calculation based on the Density Functional Theory.

### Description of Embodiments

### Alkane dehydrogenation catalyst

An alkane dehydrogenation catalyst according to an embodiment of the present disclosure contains a graphene having at least one type of structure selected from an atomic vacancy structure, a singly hydrogenated vacancy structure, a doubly hydrogenated vacancy structure, a triply hydrogenated vacancy structure, and a nitrogen-substituted vacancy structure (preferably a graphene having a structure including an atomic vacancy). Furthermore, the alkane dehydrogenation catalyst, the atomic vacancy structure, the singly hydrogenated vacancy structure, the doubly hydrogenated vacancy structure, the triply hydrogenated vacancy structure, or the nitrogen-substituted vacancy structure of graphene acts as an activation point.

Typically, a catalyst includes a metal as an active ingredient; however, when the alkane dehydrogenation catalyst includes a graphene having at least one type of structure selected from an atomic vacancy structure, a singly hydrogenated vacancy structure, a doubly hydrogenated vacancy structure, a triply hydrogenated vacancy structure, and a nitrogen-substituted vacancy structure, such a structure acts as an activation point, rendering the inclusion of metal unnecessary. The alkane dehydrogenation catalyst may optionally include a metal; a content of the metal may be, for example, 1 wt.% or less, 0.1 wt.% or less, or 0.01 wt.% or less of the content of the graphene, or may be substantially free of metal.

The alkane dehydrogenation catalyst may contain a graphene having at least one type of structure selected from an atomic vacancy structure, a singly hydrogenated vacancy structure, a doubly hydrogenated vacancy structure, a triply hydrogenated vacancy structure, and a nitrogen-substituted vacancy structure; however, from a viewpoint of achieving a better catalytic effect or an effect of lowering activation barrier, it is preferable to select and use a structure based on the application.

For example, when the primary intention is to lower the activation barrier of a hydrogen adsorption-storage reaction with an alkane, it is preferable to use a graphene containing at least an atomic vacancy structure as a catalyst. This is because a graphene containing at least an atomic vacancy structure has outstanding hydrogen adsorption capacity, leading to outstanding effect of lowering the activation barrier.

When the primary intention is to lower the activation barrier of a reaction of extracting hydrogen atoms from an alkane, it is preferable to use a graphene containing at least a nitrogen-substituted vacancy structure as a catalyst. This is because a graphene containing at least a nitrogen-substituted vacancy structure has the effect of rendering a reaction of extracting hydrogen atoms from an alkane into multiple stages and lowering the activation barrier in each of the stages.

Furthermore, to repeatedly perform a cycle of hydrogen adsorption-storage and release from an alkane, it is preferable to use a graphene containing at least a doubly hydrogenated vacancy structure or a triply hydrogenated vacancy structure as a catalyst. This is because when a graphene containing at least a doubly hydrogenated vacancy structure or a triply hydrogenated vacancy structure is used as a catalyst, although energy is required to extract one hydrogen atom from an alkane, the destabilized alkane from which one hydrogen atom is extracted spontaneously decomposes and releases hydrogen thereafter. As such, the activation layer barrier of the adsorption-storage and release reaction of hydrogen can be lowered, and the cycle can be carried out smoothly without requiring a large amount of energy.

According to the alkane dehydrogenation catalyst, the activation barrier associated with the dehydrogenation reaction of an alkane can be lowered, and hydrogen can be efficiently extracted from an alkane under mild conditions without the generation of CO₂. Furthermore, the hydrogen extracted from an alkane can be stored in the alkane dehydrogenation catalyst, and the stored hydrogen can be released as needed.

### Graphene having atomic vacancy

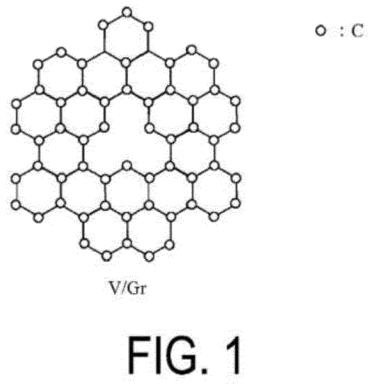

A graphene having an atomic vacancy according to an embodiment of the present disclosure (may be referred to as a "V/graphene" in the present specification) is a graphene, which is a two-dimensional atomic film in which sp² carbon atoms are bonded in a honeycomb lattice pattern, having a structure in which a hole is formed because of a carbon atom vacancy at a certain spot, that is, one carbon atom which should have been present in a complete honeycomb structure being missing (see FIG. 1).

Furthermore, the atomic vacancy structure (sometimes referred to as a "V structure" in the present specification) of the V/graphene acts as an activation point (specifically, acts to lower the activation barrier during an adsorption-storage reaction and/or release reaction of hydrogen).

The V/graphene preferably has, for example, from 2 to 200 (especially, from 50 to 150, particularly from 70 to 120) V structures per 100 nm² of the atomic film of the graphene. When the presence of the V structure is too small or excessive, the effect of the invention according to an embodiment of the present disclosure tends to be difficult to achieve.

There are two types of end structures of the V/graphene, a zigzag-type structure and an armchair-type structure, and the V/graphene may have either structure. Furthermore, the V/graphene may have one or two or more substituents on an end. Examples of the substituent include a hydrogen atom, a halogen atom, a C₁₋₂₀ alkyl group, and an oxygen-containing functional group.

A graphene constituting the V/graphene is preferably a thin film graphene having a large area (for example, a large area of 10 nm² or greater, preferably 100 nm² or greater), such as a graphene contained in an epitaxial graphene or soot (or graphite) obtained by a detonation method, from a viewpoint of stabilizing and improving resistance to heat while sufficiently increasing the surface area including the catalytic activation points per unit weight of the catalyst or the specific surface area.

### Graphene having singly, doubly, or triply hydrogenated vacancy

A graphene having a singly, doubly, or triply hydrogenated vacancy according to an embodiment of the present disclosure is a graphene, which is a two-dimensional atomic film in which sp² carbon atoms are bonded in a honeycomb lattice pattern, having a structure in which a hole is formed because of a carbon atom vacancy at a certain spot, that is, one carbon atom which should have been present in a complete honeycomb structure being missing, and from 1 to 3 hydrogen atoms are bonded to the sp² carbon atoms surrounding the hole.

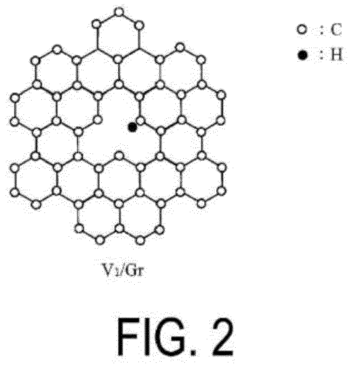

More specifically, the graphene having a singly hydrogenated vacancy (may be referred to as a "V₁/graphene" in the present specification) is a graphene, which is a two-dimensional atomic film in which sp² carbon atoms are bonded in a honeycomb lattice pattern, having a structure in which a hole is formed because of a carbon atom vacancy at a certain spot, that is, one carbon atom which should have been present in a complete honeycomb structure being missing, and one hydrogen atom is bonded to any one of the sp² carbon atoms surrounding the hole (see FIG. 2).

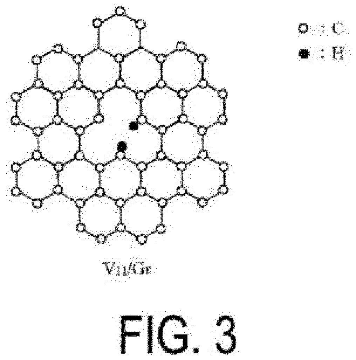

The graphene having a doubly hydrogenated vacancy (may be referred to as a "V₁₁/graphene" in the present specification) is a graphene, which is a two-dimensional atomic film in which sp² carbon atoms are bonded in a honeycomb lattice pattern, having a structure in which a hole is formed because of a carbon atom vacancy at a certain spot, that is, one carbon atom which should have been present in a complete honeycomb structure being missing, and two hydrogen atoms are separately bonded to any two of the sp² carbon atoms surrounding the hole (see FIG. 3).

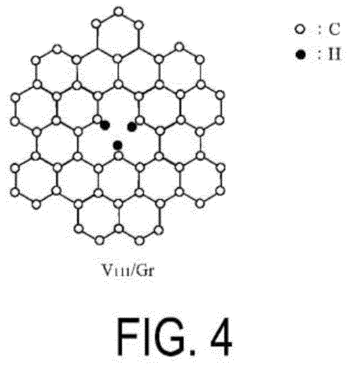

The graphene having a triply hydrogenated vacancy (may be referred to as a "V₁₁₁/graphene" in the present specification) is a graphene, which is a two-dimensional atomic film in which sp² carbon atoms are bonded in a honeycomb lattice pattern, having a structure in which a hole is formed because of a carbon atom vacancy at a certain spot, that is, one carbon atom which should have been present in a complete honeycomb structure being missing, and three hydrogen atoms are separately bonded to any three of the sp² carbon atoms surrounding the hole (see FIG. 4).

Furthermore, the singly hydrogenated vacancy structure (may be referred to as a "V₁ structure" in the present specification) of the V₁/graphene acts as an activation point.

The doubly hydrogenated vacancy structure (may be referred to as a "V₁₁ structure" in the present specification) of the V₁₁/graphene acts as an activation point.

The triply hydrogenated vacancy structure (may be referred to as a "V₁₁₁ structure" in the present specification) of the V₁₁₁/graphene acts as an activation point.

Note that "acting as an activation point" means to lower the activation barrier during an adsorption-storage reaction and/or release reaction of hydrogen.

A structure selected from the V₁ structure, V₁₁ structure and V₁₁₁ structure is preferably present in the graphene having a singly, doubly, or triply hydrogenated vacancy in a number from 2 to 200 (especially from 50 to 150, particularly from 70 to 120) per 100 nm² of the atomic film of the graphene. When the presence of the structure selected from the V₁ structure, V₁₁ structure and V₁₁₁ structure is too small or excessive, the effect of the invention according to an embodiment of the present disclosure tends to be difficult to achieve.

There are two types of end structures of the graphene having a singly, doubly, or triply hydrogenated vacancy, a zigzag-type structure and an armchair-type structure, and the graphene having a singly, doubly, or triply hydrogenated vacancy may have either structure. Furthermore, the graphene having a singly, doubly, or triply hydrogenated vacancy may have one or two or more substituents on an end. Examples of the substituent include a hydrogen atom, a halogen atom, a C₁₋₂₀ alkyl group, and an oxygen-containing functional group.

A graphene constituting the graphene having a singly, doubly, or triply hydrogenated vacancy is preferably a thin film graphene having a large area (for example, a large area of 10 nm² or greater, preferably 100 nm² or greater), such as a graphene contained in an epitaxial graphene or soot (or graphite) obtained by a detonation method, from a viewpoint of stabilizing and improving resistance to heat while sufficiently increasing the surface area including the catalytic activation points per unit weight of the catalyst or the specific surface area.

### Graphene having nitrogen-substituted vacancy

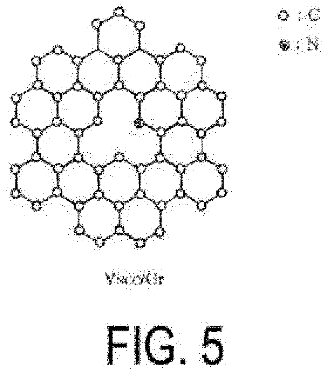

A graphene having a nitrogen-substituted vacancy (may be referred to as a "V_{N}/graphene" in the present specification) is a graphene, which is a two-dimensional atomic film in which sp² carbon atoms are bonded in a honeycomb lattice pattern, having a structure (may be referred to as a "V_{N} structure" in the present specification) in which a hole is formed because of a carbon atom vacancy at a certain spot, that is, one carbon atom which should have been present in a complete honeycomb structure being missing, and any one of the 12 sp² carbon atoms surrounding the hole, or any one of the sp² carbon atoms present in the vicinity of the hole, is substituted with a nitrogen atom (see FIG. 5).

The local arrangement of nitrogen and carbon of the V_{N} structure is, for example, a ketoimine structure, an imine structure, and a flat carbon nitride structure (that is, graphitic carbon nitride). The V_{N}/graphene may have one V_{N} structure selected from the structures, or may have two or more V_{N} structures.

A content of nitrogen atoms in the V_{N}/graphene is, for example, from 100 ppm to 7 wt.% of the total amount of the V_{N}/graphene, preferably from 3 to 5 wt.% from a viewpoint of further lowering the activation barrier associated with the dehydrogenation reaction of an alkane. When the presence of the V_{N} structure is too small or excessive, the effect of the invention according to an embodiment of the present disclosure tends to be difficult to achieve.

Furthermore, the nitrogen-substituted vacancy structure (sometimes referred to as a "V_{NCC} structure" in the present specification) of the V_{N}/graphene acts as an activation point (specifically, acts to lower the activation barrier during an adsorption-storage reaction and/or release reaction of hydrogen).

The V_{N}/graphene preferably has, for example, from 2 to 200 (especially, from 50 to 150, particularly from 70 to 120) V_{NCC} structures per 100 nm² of the atomic film of the graphene. When the presence of the V_{NCC} structure is too small or excessive, the effect of the invention according to an embodiment of the present disclosure tends to be difficult to achieve.

There are two types of end structures of the V_{N}/graphene, a zigzag-type structure and an armchair-type structure, and the V_{N}/graphene may have either structure. Furthermore, the V_{N}/graphene may have one or two or more substituents on an end. Examples of the substituent include a hydrogen atom, a halogen atom, a C₁₋₂₀ alkyl group, and an oxygen-containing functional group.

A graphene constituting the V_{N}/graphene is preferably a thin film graphene having a large area (for example, a large area of 10 nm² or greater, preferably 100 nm² or greater), such as a thin film graphene isolated from an epitaxial graphene or graphite obtained by a detonation method, from a viewpoint of stabilizing and improving resistance to heat while sufficiently increasing the surface area including the catalytic activation points per unit weight of the catalyst or the specific surface area.

Method of producing alkane dehydrogenation catalyst

The alkane dehydrogenation catalyst described above can be produced through a step of colliding high-energy particles with a raw material graphene.

The alkane dehydrogenation catalyst is preferably produced through the following steps.
Step A: Producing a raw material graphene
Step B: Colliding high-energy particles (such as electrons and ions) with the resulting graphene

### Method of producing alkane dehydrogenation catalyst containing V/graphene

An alkane dehydrogenation catalyst containing V/graphene can be produced, for example, through the following steps.
Step A: Producing a raw material graphene
Step B: Colliding high-energy particles with the resulting graphene to obtain a graphene having an atomic vacancy (that is, V/graphene)

Step A is a step of producing a raw material graphene, that is, the graphene serving as a raw material of the alkane dehydrogenation catalyst. The raw material graphene can be produced by a variety of methods; among the graphenes produced by a variety of methods, it is preferable to use at least one selected from an epitaxial graphene and a chemically synthesized single-layer nanographene, from a viewpoint of increasing the specific surface area which in turn maximizes the area of contact between an activation point of the catalyst and the alkane in the gas phase or in the liquid phase.

In addition, a graphene obtained by a CVD method, in which a hydrocarbon such as methane is heated in the presence of a metal catalyst, may be used.

Furthermore, some thin film graphenes (to be described in detail later) that can be obtained by a detonation method may contain nitrogen, and these thin film graphenes can also be used as a raw material graphene of V/graphene or V₁/graphene.

The epitaxial graphene can be synthesized, for example, by thermally decomposing a SiC substrate (for example, heating at an ultra-high temperature of approximately 2150°C).

Examples of the high-energy particles used in Step B include, for example, electrons and ions.

As a method for colliding electrons with graphene, a method of directly irradiating graphene with electron beams, or an internal electron irradiation method utilizing the Compton effect by gamma ray irradiation can be adopted.

The ions are preferably an ionized inert gas such as argon gas, neon gas, helium gas, xenon gas, krypton gas, and nitrogen gas.

Examples of a method of colliding ions with graphene include, for example, a method of knocking out a carbon atom constituting a graphene (ion sputtering method), which includes introducing a small amount of an inert gas to a vacuum vessel (with a degree of vacuum of, for example, from 0.1 × 10⁻⁵ to 1.5 × 10⁻⁵ Pa) having a grid electrode electrically insulated from a high melting point transition metal filament heated to approximately from 1000 to 2500°C, ionizing the inert gas by thermions generated by applying a voltage of, for example, from 0 to 500 V, between the filament and the grid electrode, and applying a high voltage (for example, from 0.02 to 4.0 kV) between the target graphene and the electrode to cause the ionized inert gas to collide with the surface of the target graphene at a high speed (with a time of ion collision of, for example, from 1 to 60 minutes).

By colliding high-energy particles with a graphene, a carbon atom at any position can be knocked out from the graphene structure while essentially maintaining the structure of the graphene, forming an atomic vacancy.

### Method of producing alkane dehydrogenation catalyst containing V₁/graphene

An alkane dehydrogenation catalyst containing V₁/graphene can be produced, for example, through the following steps.
Step A: Producing a raw material graphene
Step B: Colliding high-energy particles with the resulting graphene to obtain a graphene having an atomic vacancy (that is, V/graphene)
Step C: Hydrogenating the graphene having an atomic vacancy (that is, V/graphene) to obtain a graphene having a singly hydrogenated vacancy (that is, a V₁/graphene)

Steps A and B can employ the same method as the method of producing an alkane dehydrogenation catalyst containing V/graphene.

Hydrogenation of V/graphene in Step C can be carried out, for example, by reacting the V/graphene with hydrogen gas (molecular hydrogen).

A hydrogen partial pressure at the time of hydrogenation is, for example, approximately from 10⁻⁷ to 2 atmospheres at ambient temperature. As such, a hydrogen atom is introduced to an atomic vacancy site of the graphene, resulting in a V₁/graphene.

The alkane dehydrogenation catalyst containing V₁/graphene can also be produced through the following steps.
Step A: Producing a raw material graphene
Step D: Colliding hydrogen ions with the resulting raw material graphene to perform formation of atomic vacancy and hydrogenation at the same time to give a V₁/graphene

Steps A can employ the same method as the method of producing an alkane dehydrogenation catalyst containing V/graphene.

The method of colliding hydrogen ions with the raw material graphene in Step D can be performed by reacting the raw material graphene with atomic hydrogen that is generated by contacting molecular hydrogen with a high melting point transition metal filament heated to approximately from 1000 to 2500°C.

### Method of producing alkane dehydrogenation catalyst containing V₁₁/graphene

An alkane dehydrogenation catalyst containing V₁₁/graphene can be obtained by, for example, colliding hydrogen ions with a raw material graphene to perform formation of atomic vacancy and hydrogenation at the same time to give a V₁₁/graphene. In addition, the alkane dehydrogenation catalyst containing V₁₁/graphene can also be produced by hydrogenating the V/graphene or V₁/graphene.

### Method of producing alkane dehydrogenation catalyst containing V₁₁₁/graphene

An alkane dehydrogenation catalyst containing V₁₁₁/graphene can be obtained by, for example, colliding hydrogen ions with a raw material graphene to perform formation of atomic vacancy and hydrogenation at the same time to give a V₁₁₁/graphene. In addition, the alkane dehydrogenation catalyst containing V₁₁₁/graphene can also be produced by hydrogenating the V/graphene, V₁/graphene, or V₁₁/graphene.

### Method of producing alkane dehydrogenation catalyst containing V_{N}/graphene

An alkane dehydrogenation catalyst containing V_{N}/graphene can be produced, for example, through the following steps.
Step A: Producing a raw material graphene
Step E: Colliding nitrogen ions with the resulting raw material graphene to perform formation of atomic vacancy and nitrogenation at the same time to give a graphene having a nitrogen-substituted vacancy (that is, V_{N}/graphene)

Steps A can employ the same method as the method of producing an alkane dehydrogenation catalyst containing V/graphene.

The method of colliding nitrogen ions with the resulting raw material graphene in Step E can be, for example, one in which nitrogen beam source is used as an ion source of an ion sputtering apparatus with the acceleration voltage set to approximately from 0.1 to 1 MeV (preferably from 0.18 to 0.22 MeV), and nitrogen substitution is performed at the same time as vacancy formation by irradiating the raw material graphene with nitrogen beams. Note that the raw graphene may be exposed to nitrogen gas at the time of or after irradiation with nitrogen beams.

Furthermore, the alkane dehydrogenation catalyst containing V_{N}/graphene can also be produced through the following steps.
Step A': Producing a nitrogen-containing raw material graphene
Step F: Colliding high-energy particles with the resulting nitrogen-containing raw material graphene to obtain a graphene having a nitrogen-substituted vacancy (that is, V_{N}/graphene)

Step A' is a step of producing a nitrogen-containing raw material graphene. The nitrogen-containing raw material graphene is preferably a graphene obtained by a detonation method (more specifically, a thin film graphene contained in soot, or graphite, obtained by a detonation method) or a CVD synthesized graphene, from a viewpoint of having a large specific surface area to maximize the area of contact between an activation point of the catalyst and the alkane in the gas phase or in the liquid phase.

Using a detonation method, a graphene can be produced through the following steps.
[1] An explosive primed with an electric detonator is placed inside a pressure-resistant detonation vessel, and the vessel is sealed in a state where a gas of atmospheric composition at normal pressure and the explosive to be used coexist inside the vessel. The vessel is made of, for example, iron and has a capacity of, for example, from 0.1 to 40 m³. A mixture of trinitrotoluene (TNT) and cyclotrimethylenetrinitramine, i.e., hexogen (RDX), can be used as the explosive. The mass ratio (TNT/RDX) of TNT to RDX is, for example, in a range from 40/60 to 60/40.
[2] Next, the electric detonator is triggered to detonate the explosive in the vessel. During detonation, the explosive that is used undergoes partially incomplete combustion and releases carbon, which serves as a raw material for generating graphite.
[3] Next, the vessel and the content of the vessel are left to stand for approximately 24 hours at room temperature, and thus, are cooled. After the cooling, the graphite containing impurities (that is, crude graphite) deposited on the inner wall of the vessel is scraped with a spatula and collected.
[4] Next, the collected crude graphite is subjected to a purification treatment to obtain a purified graphite. The purification treatment is preferably performed by a method in which the crude graphite is stirred and washed with a washing solution [water or acidic dispersion solution (for example, hydrochloric acid, nitric acid, sulfuric acid) and then removed from the washing solution and dried. The drying temperature can be selected as appropriate in a range from room temperature to 1500°C. After drying, the product may also be annealed at a temperature from room temperature to 1500°C for from 1 minute to 5 hours.

The purified graphite is a combination of multiple graphenes held by van der Waals forces, from which graphene can be isolated. As a method of isolating graphene, a well-known and commonly used method such as a method of peeling on a silicon oxide surface or a method of cutting and peeling by a mechanical method (such as a milling method) can be adopted.

The CVD synthesized graphene can be produced, for example, by a method of heating a hydrocarbon, such as methane, and ammonia in the presence of a metal catalyst (CVD method).

Step F can be performed in the same manner as in Step B, except that the nitrogen-containing raw material graphene is used instead of a graphene.

A nitrogen content of the nitrogen-containing raw material graphene is, for example, in a range from 1 ppm to 50 wt.%, of which a range from 1 to 10 wt.% is preferable from a viewpoint of increasing the catalytic effect of the resulting alkane dehydrogenation catalyst.

In addition to the nitrogen-containing raw material graphene, a raw material that can be used to produce the alkane dehydrogenation catalyst containing V_{N}/graphene include: a raw material graphene having a nitro group, an amide group, an oxime structure, or a nitrile structure; a raw material nanographene having a group or a structure described above; and a polycyclic aromatic compound having a group or a structure described above.

### Method of producing hydrogen

The method of producing hydrogen according to an embodiment of the present disclosure includes a step of extracting hydrogen from an alkane using the alkane dehydrogenation catalyst.

In the step described above, the hydrogen extracted from an alkane is adsorbed and stored in an atomic vacancy site of the alkane dehydrogenation catalyst. The adsorbed and stored hydrogen can then be released from the alkane dehydrogenation catalyst as needed.

As such, the method of producing hydrogen preferably includes the following steps.
Step (1): Extracting hydrogen from an alkane using the alkane dehydrogenation catalyst and storing the hydrogen (hydrogen adsorption-storage step)
Step (2): Releasing the hydrogen from the alkane dehydrogenation catalyst (hydrogen release step)

For the alkane, which is the raw material alkane, for example, an alkane having from 3 to 25 carbons can be used. Specific examples of the alkane include: a linear or branched alkane, such as n-propane, n-butane, isobutane, n-pentane, n-hexane, n-heptane, n-octane, 3-methylheptane, n-nonane, and a paraffin; and a cycloalkane, such as cyclopropane, cyclopentane, cyclohexane, and cyclooctane. One of these can be used alone or two or more in combination. Furthermore, the raw material alkane can contain a component in addition to the alkane.

An amount of the alkane dehydrogenation catalyst to be used is, for example, approximately from 0.0001 to 1 parts by weight, preferably from 0.01 to 0.25 parts by weight, per 100 parts by weight of the alkane.

For example, when a V/graphene, V₁/graphene, V₁₁/graphene, or V_{N}/graphene is used as the alkane dehydrogenation catalyst, a large amount of hydrogen can be adsorbed to and stored in the catalyst by the proceeding of the following reactions during the aforementioned Step (1) (hydrogen adsorption-storage step).

Step (1)-1: A hydrogen atom site on an alkane is adsorbed to an atomic vacancy site on the graphene, two hydrogen atoms are extracted from the alkane, and the two extracted hydrogen atoms are incorporated into the atomic vacancy site

Step (1)-2: The hydrogen atoms that are incorporated into the atomic vacancy site are diffused from the site to another site on the graphene and are stored in the other site

For example, when a V/graphene is used as the alkane dehydrogenation catalyst, in Step (1)-1, a hydrogen atom site on an alkane is adsorbed to an atomic vacancy site having a V structure of the graphene, and two hydrogen atoms of the alkane are incorporated into the atomic vacancy site. As such, the structure of the atomic vacancy site changes from an atomic vacancy structure (V structure) to a doubly hydrogenated vacancy structure (V₁₁ structure).

In Step (1)-2, in some of the doubly hydrogenated vacancy structures (V₁₁ structures) generated, the two hydrogen atoms present at the atomic vacancy site move from the atomic vacancy site to another site of the graphene as a result of a surface diffusion reaction (migration), and are adsorbed on a carbon atom at the destination of the movement. As a result, the structure of the atomic vacancy site returns to an atomic vacancy structure from a doubly hydrogenated vacancy structure.

Furthermore, other doubly hydrogenated vacancy structures generated (V₁₁ structures) undergo changes as in a case in which V₁₁/graphene is used as the alkane dehydrogenation catalyst as described below.

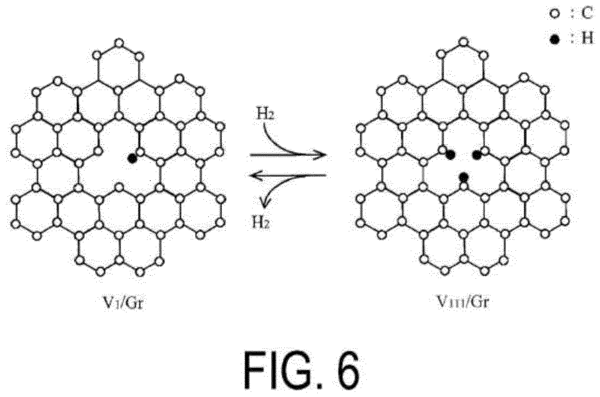

For example, when a V₁/graphene is used as the alkane dehydrogenation catalyst, in Step (1)-1, a hydrogen atom site on an alkane is adsorbed to an atomic vacancy site having a V₁ structure of the graphene, and two hydrogen atoms of the alkane are incorporated into the atomic vacancy site. As such, the structure of the atomic vacancy site changes from a singly hydrogenated vacancy structure (V₁ structure) to a triply hydrogenated vacancy structure (V₁₁₁ structure) (FIG. 6).

In Step (1)-2, in some of the triply hydrogenated vacancy structures (V₁₁₁ structures) generated, two of the three hydrogen atoms present at the atomic vacancy site then move from the atomic vacancy site to another site as a result of a surface diffusion reaction (migration), and are adsorbed on a carbon atom at the destination of the movement. As a result, the structure of the atomic vacancy site returns to a singly hydrogenated vacancy structure from a triply hydrogenated vacancy structure.

Furthermore, other triply hydrogenated vacancy structures generated (V₁₁₁ structures) undergo changes as in a case in which V₁₁₁/graphene is used as the alkane dehydrogenation catalyst as described below.

For example, when a V₁₁/graphene is used as the alkane dehydrogenation catalyst, in Step (1)-1, a hydrogen atom site on an alkane is adsorbed to an atomic vacancy site having a V₁₁ structure of the graphene, and two hydrogen atoms of the alkane are incorporated into the atomic vacancy site. As such, the structure of the atomic vacancy site changes from a doubly hydrogenated vacancy structure (V₁₁ structure) to a quadruply hydrogenated vacancy structure (V₂₁₁ structure).

Then, in Step (1)-2, two hydrogen atoms present at the atomic vacancy site move from the atomic vacancy site of the graphene to another site as a result of a surface diffusion reaction (migration), and are adsorbed on a carbon atom at the destination of the movement. As a result, the structure of the atomic vacancy site returns to a doubly hydrogenated vacancy structure from a quadruply hydrogenated vacancy structure.

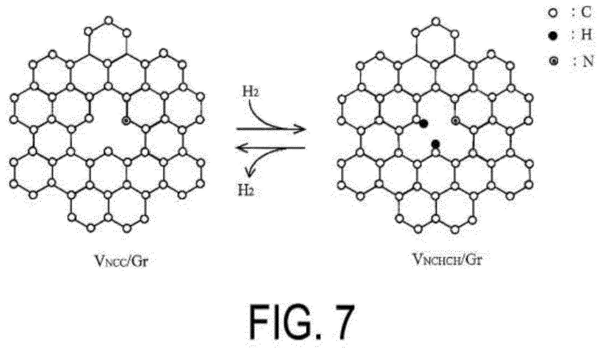

For example, when a V_{N}/graphene is used as the alkane dehydrogenation catalyst, in Step (1)-1, a hydrogen atom site on an alkane is adsorbed to an atomic vacancy site having a V_{NCC} structure of the graphene, and two hydrogen atoms of the alkane are incorporated into the atomic vacancy site. As such, the structure of the atomic vacancy site changes from a nitrogen-substituted vacancy structure (V_{NCC} structure) to a doubly hydrogenated nitrogen-substituted vacancy structure (V_{NCHCH} structure) (FIG. 7).

Then, in Step (1)-2, the two hydrogen atoms present at the atomic vacancy site move from the atomic vacancy site to another site as a result of a surface diffusion reaction (migration), and are adsorbed on a carbon atom at the destination of the movement. As a result, the structure of the atomic vacancy site returns to a nitrogen-substituted vacancy structure from a doubly hydrogenated nitrogen-substituted vacancy structure.

When the structure of the atomic vacancy site of the graphene is restored in Step (1)-2 as described above, the reaction, that is, the hydrogen adsorption-storage reaction, proceeds again in the order from Step (1)-1 to Step (1)-2. As the hydrogen adsorption-storage reaction proceeds continuously, a large amount of hydrogen atoms can be extracted from the alkane and stored in the alkane dehydrogenation catalyst.

An amount of hydrogen atoms that can be stored is, for example, 10 or more such as from 10 to 30, preferably 20 or more such as from 20 to 30, per atomic vacancy of graphene. Furthermore, the amount of hydrogen atom that can be stored is, for example, 1.0 × 10¹⁶, preferably from 1.0 × 10¹⁶ to 1.5 × 10¹⁶, per 1 cm² of the V₁/graphene or the V_{N}/graphene.

Furthermore, the hydrogen atoms adsorbed to and stored in the alkane dehydrogenation catalyst in Step (1) can be released from the alkane dehydrogenation catalyst as a hydrogen molecule that is formed by two stored hydrogen atoms joining together; this is a result of Step (2), which is performing the reaction of Step (1) in a reverse order, first Step (1)-2 then Step (1)-1.

For example, when a V₁/graphene is used as the alkane dehydrogenation catalyst, two of the three hydrogen atoms present in an atomic vacancy site of a triply hydrogenated vacancy, formed as a result of adsorption and storage of hydrogen atoms, can be joined to form a hydrogen molecule, and the hydrogen molecule formed can be released to the outside. Note that, although the atomic vacancy is restored to a singly hydrogenated vacancy after the hydrogen molecule is released, when hydrogen atoms move to the vacancy as a result of migration, a triply hydrogenated vacancy is formed again, and the reaction described above proceeds. Then, as the reaction proceeds continuously, a large amount of hydrogen molecules can be released from the alkane dehydrogenation catalyst.

When a V_{N}/graphene is used as the alkane dehydrogenation catalyst, two hydrogen atoms present in an atomic vacancy site of a doubly hydrogenated nitrogen-substituted vacancy, formed as a result of adsorption and storage of hydrogen atoms, can be joined to form a hydrogen molecule, and the hydrogen molecule formed can be released to the outside. Note that, although the atomic vacancy is restored to a nitrogen-substituted vacancy after the hydrogen molecule is released, when hydrogen atoms move to the vacancy as a result of migration, a doubly hydrogenated nitrogen-substituted vacancy is formed again, and the reaction described above proceeds. Then, as the reaction proceeds continuously, a large amount of hydrogen molecules can be released from the alkane dehydrogenation catalyst.

As a result of using at least one selected from V/graphene, V₁/graphene, V₁₁/graphene, and V_{N}/graphene as the alkane dehydrogenation catalyst to extract hydrogen from an alkane, the raw material alkane is decomposed, and, through an intermediate (a compound with an unbonded bond that gives a lone pair of electrons), a small alkane and an alkyne are generated. For example, an n-octane represented by Formula (P1) below is used as a raw material, and two hydrogen atoms at a site indicated by the surrounding dotted line are extracted from the n-octane; as a result, mainly, through an intermediate represented by Formula (P2) below, an n-pentane represented by Formula (P3) below and a propyne represented by Formula (P4) below are generated.

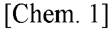

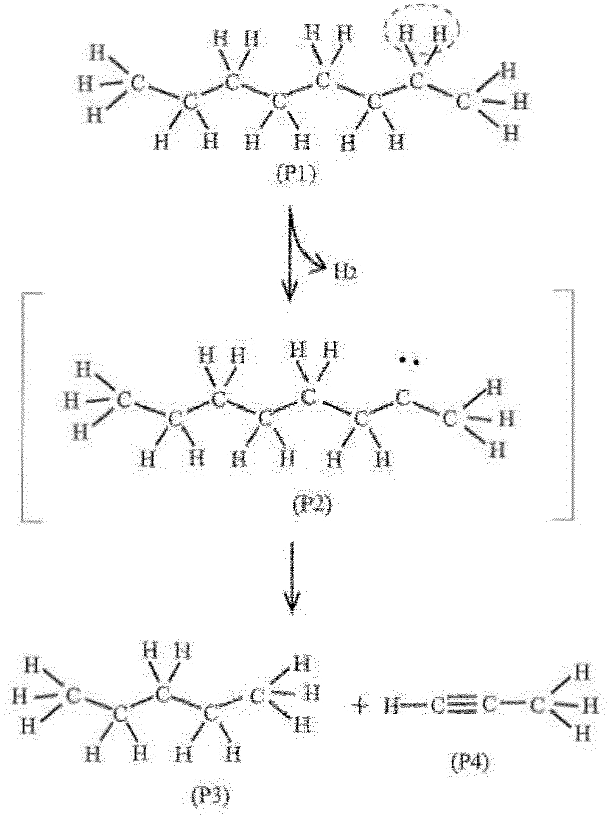

Therefore, when V₁/graphene is used as the alkane dehydrogenation catalyst to extract hydrogen from n-octane, the following reaction proceeds during Step (1) (hydrogen adsorption-storage step), resulting in hydrogen along with n-pentane and propyne. Furthermore, as is clear from the following reaction formula, CO₂ is not generated during this step.

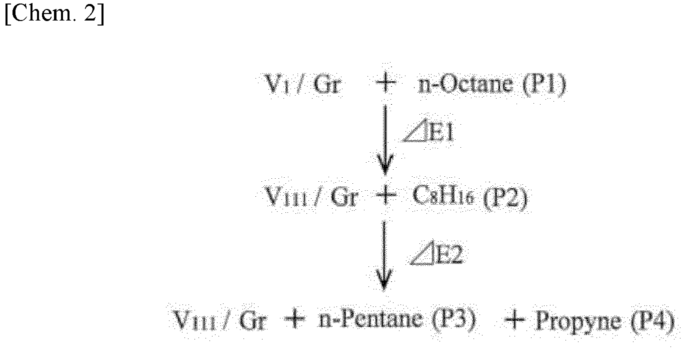

When a V₁/graphene is used as the alkane dehydrogenation catalyst, the activation barrier of hydrogen adsorption-storage reaction is dramatically lower compared to when a graphene without an atomic vacancy site is used. The ΔE1 is, for example, approximately 3.1 eV, and the ΔE2 is, for example, approximately 1.6 eV.

When a V₁/graphene is used as the alkane dehydrogenation catalyst, the hydrogen adsorption-storage reaction can proceed, for example, by heating to approximately from 450 to 750°C.

Furthermore, when a V₁/graphene is used as the alkane dehydrogenation catalyst, hydrogen stored in the alkane dehydrogenation catalyst can be released in Step (2) (hydrogen release step) without requiring a significant amount of energy because of the following reactions.

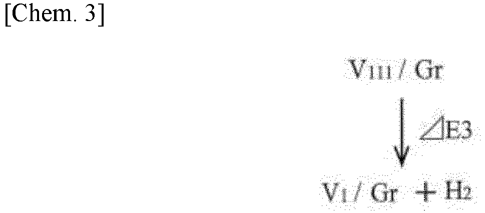

The ΔE3 is, for example, approximately 4.7 eV. The hydrogen release reaction can proceed, for example, by heating to approximately from 680 to 1200°C.

Furthermore, when a V_{N}/graphene containing a nitrogen having a high affinity for hydrogen is used as the alkane dehydrogenation catalyst to extract hydrogen from n-octane, the following reaction proceeds during Step (1) (hydrogen adsorption-storage step), resulting in hydrogen along with n-pentane and propyne as the reaction products. When a V_{N}/graphene is used as the alkane dehydrogenation catalyst, the reaction contains multiple stages compared to when a V₁/graphene is used as the alkane dehydrogenation catalyst. As such, the activation barrier at each stage is lower than when a V₁/graphene is used as the alkane dehydrogenation catalyst. This allows the reaction to proceed under milder conditions than when a V₁/graphene is used as the alkane dehydrogenation catalyst.

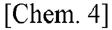

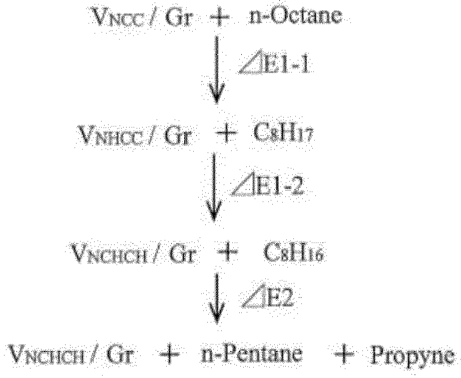

When a V_{N}/graphene is used as the alkane dehydrogenation catalyst, the ΔE1-1 is, for example, approximately 1.7 eV, and the ΔE1-2 is, for example, approximately 1.7 eV.

When a V_{N}/graphene is used as the alkane dehydrogenation catalyst, the hydrogen adsorption-storage reaction can proceed, for example, by heating to approximately from 300 to 500°C.

Meanwhile, when a V_{N}/graphene is used as the alkane dehydrogenation catalyst, the following ΔE3 related to the hydrogen release reaction is, for example, approximately 4.7 eV. Therefore, the reaction can proceed by heating to, for example, approximately from 680 to 1200°C.

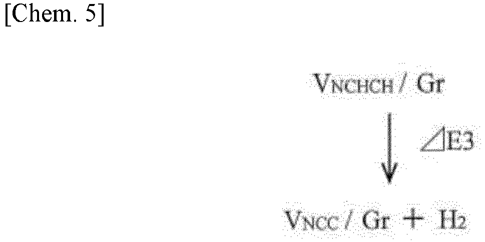

The reaction pressure at this time is, for example, approximately from 100 to 1500 kPa. Furthermore, the reaction atmosphere of the reaction above is not particularly limited as long as it does not inhibit the reaction. For example, an air atmosphere, a nitrogen atmosphere, or an argon atmosphere may be used.

For example, when a V₁₁/graphene and/or a V₁₁₁/graphene is used as the alkane dehydrogenation catalyst, a large amount of hydrogen can be adsorbed to and stored in the catalyst by the proceeding of the following reactions during the aforementioned Step (1) (hydrogen adsorption-storage step).

Hereinafter, a case where V₁₁₁/graphene is used as the alkane dehydrogenation catalyst will be described in detail. When a V₁₁/graphene is used as the alkane dehydrogenation catalyst, the description above applies except that the V₁₁ structure changes to a V₁₁₁ structure.

Step (1)-11: A hydrogen atom site on an alkane is adsorbed to an atomic vacancy site having a V₁₁₁ structure on the graphene, one hydrogen atom is extracted from the alkane, and the extracted hydrogen atom is incorporated into the atomic vacancy site having a V₁₁₁ structure. As such, the structure of the atomic vacancy site changes from a triply hydrogenated vacancy structure (V₁₁₁ structure) to a quadruply hydrogenated vacancy structure (V₂₁₁ structure).

Step (1)-12: The alkane with one hydrogen atom extracted spontaneously decomposes and, through an intermediate, generates a small alkane and an alkyne, during which one hydrogen atom is released. The released hydrogen atom is adsorbed on and stored in a carbon atom at a site other than the atomic vacancy site of graphene.

Step (1)-13: One of the four hydrogen atom present at an atomic vacancy site moves from the atomic vacancy site to another site as a result of a surface diffusion reaction (migration), and is adsorbed on a carbon atom at the destination of the movement. As a result, the structure of the atomic vacancy site returns to a triply hydrogenated vacancy structure from a quadruply hydrogenated vacancy structure.

As such, when the triply hydrogenated vacancy structure (V₁₁₁ structure) of graphene is restored, the reaction (that is, hydrogen adsorption-storage reaction) proceeds again in the order from Step (1)-11 to Step (1)-12. As the hydrogen adsorption-storage reaction proceeds continuously, a large amount of hydrogen atoms can be extracted from the alkane and stored in the alkane dehydrogenation catalyst.

Furthermore, when a V₁₁₁/graphene is used as the alkane dehydrogenation catalyst to extract one hydrogen from an alkane, the alkane, which is a raw material, generates an unstable intermediate. Such unstable intermediate spontaneously decomposes, generating a small alkane and an alkene from which one hydrogen is extracted, the latter in turn further releases one hydrogen and generates an alkyne.

For example, an n-octane represented by Formula (P1) below is used as a raw material, and one hydrogen atom at a site indicated by the surrounding dotted line is extracted from the n-octane; as a result, an intermediate represented by Formula (P2') is generated. Then, the intermediate represented by Formula (P2') below spontaneously decomposes, generating mainly an n-pentane, represented by Formula (P3) below, and an intermediate which is a propylene from which one hydrogen is extracted, represented by Formula (P4') below. Then, one hydrogen atom is released from the intermediate represented by Formula (P4') below, generating a propyne represented by Formula (P4) below.

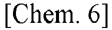

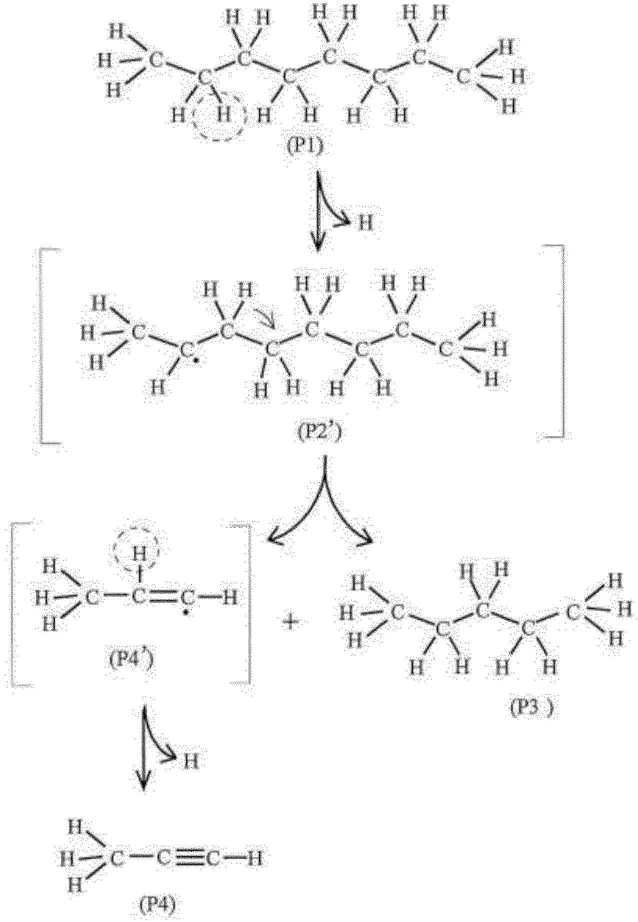

Therefore, when a V₁₁₁/graphene is used as the alkane dehydrogenation catalyst to extract hydrogen from the n-octane, the following reaction proceeds during Step (1) (hydrogen adsorption-storage step), resulting in hydrogen along with n-pentane and propyne. Furthermore, as is clear from the following reaction formula, CO₂ is not generated during this step.

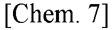

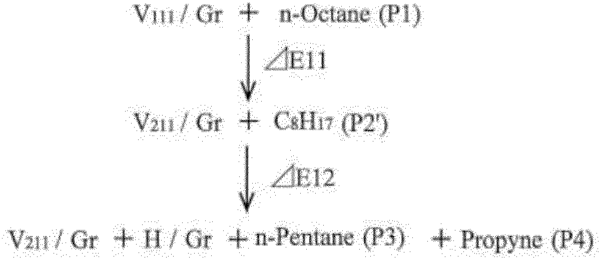

When a V₁₁₁/graphene is used as the alkane dehydrogenation catalyst, the activation barrier of hydrogen adsorption-storage reaction is dramatically lower compared to when a graphene without an atomic vacancy site is used as the catalyst. The ΔE11 is, for example, approximately 4.0 eV, and the ΔE12 is, for example, approximately 3.3 eV.

When a V₁₁₁/graphene is used as the alkane dehydrogenation catalyst, the hydrogen adsorption-storage reaction can proceed, for example, by heating to approximately from 570 to 950°C.

Furthermore, when a V₁₁₁/graphene is used as the alkane dehydrogenation catalyst, hydrogen stored in the alkane dehydrogenation catalyst can be released in Step (2) (hydrogen release step) without requiring a significant amount of energy because of the following reactions.

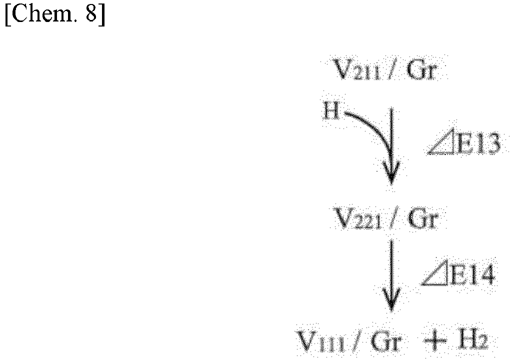

Step (2)-1: A hydrogen atom moves from a site other than an atomic vacancy site of the graphene to an atomic vacancy site having a V₂₁₁ structure of the graphene because of migration. As such, the structure of the atomic vacancy site changes from a V₂₁₁ structure to a V₂₂₁ structure (quintuply hydrogenated deficiency structure).

Step (2)-2: Two of the five hydrogen atoms present in the atomic vacancy site of the graphene join together to form a hydrogen molecule, which is released to the outside.

Although the structure of the atomic vacancy site returns to a V₁₁₁ structure after the hydrogen molecular is released, when hydrogen atoms move to the atomic vacancy site having a V₁₁₁ structure as a result of migration, the structure of the atomic vacancy site changes from a V₁₁₁ structure to a V₂₁₁ structure, and the release reaction proceeds again. Then, as the reaction proceeds continuously, a large amount of hydrogen molecules can be released from the alkane dehydrogenation catalyst.

The ΔE13 is, for example, approximately 1.1 eV, and the ΔE14 is, for example, approximately 1.3 eV. The hydrogen release reaction can proceed, for example, by heating to approximately from 270 to 480°C.

Note that, from the V₁₁₁/graphene after the hydrogen molecular release, hydrogen atoms can be further released to the outside as a result of the following reaction, but the activation barrier (ΔE15 below) of the reaction described below is approximately 4.7 eV and requires heating to approximately from 680 to 1200°C.

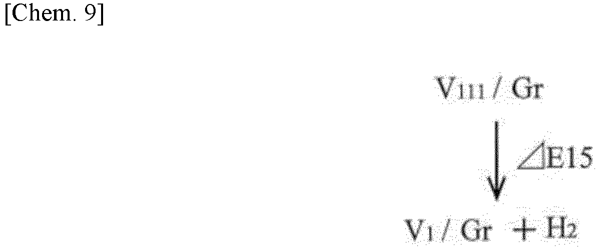

Therefore, from a viewpoint of producing, storing, and releasing hydrogen efficiently using a small amount of energy, it is preferable to extract, adsorb and store, and release hydrogen from an alkane in accordance with the cycle of V₁₁₁-V₂₁₁-V₂₂₁-V₁₁₁ described above.

The reaction pressure of the hydrogen adsorption-storage and release reaction is, for example, approximately from 1 to 1500 kPa. Furthermore, the reaction atmosphere of the reaction above is not particularly limited as long as it does not inhibit the reaction. For example, an air atmosphere, a nitrogen atmosphere, or an argon atmosphere may be used.

In addition, as shown in the following formula, a V₁₁₁/graphene also functions as a catalyst to promote dehydrogenation reaction with another V₁₁₁/graphene. This dehydrogenation reaction also generates hydrogen.

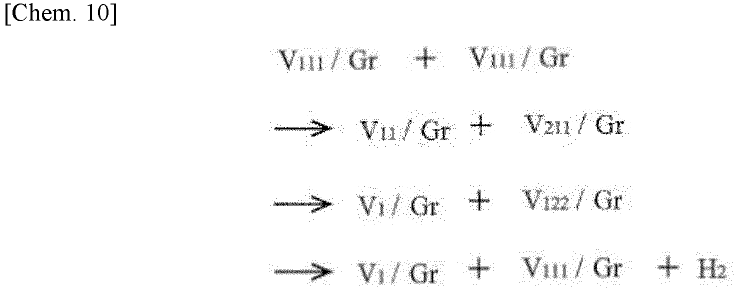

The V₁₁₁/graphene also generates hydrogen by the proceeding of the following decomposition reaction, which is a dehydrogenation reaction.

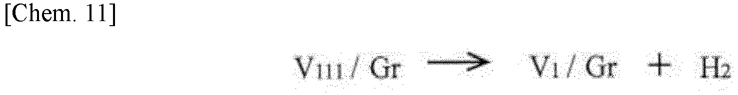

As the description above, by using the alkane dehydrogenation catalyst according to an embodiment of the present disclosure, it is possible to extract hydrogen from an alkane and release the extracted hydrogen by applying energy as appropriate.

Furthermore, although the alkane dehydrogenation catalyst described above may have a reduced catalytic effect due to the repair of an atomic vacancy site over time, the alkane dehydrogenation catalyst can be activated in such a case by colliding ions with the alkane dehydrogenation catalyst again to form an atomic vacancy. Therefore, the catalyst can be used repeatedly, which is economical.

In the method of producing hydrogen according to an embodiment of the present disclosure, hydrogen is obtained as a reaction product, along with n-pentane and propyne which are decomposition products of a raw material alkane. The reaction product can be separated using a well-known and commonly-used method, resulting in hydrogen which is useful as a renewable energy.

Regarding the hydrogen thus obtained, CO₂ is not generated during the stage of using the hydrogen as energy, nor is CO₂ generated during the stage of producing the hydrogen. As such, the hydrogen obtained by the method of producing hydrogen according to an embodiment of the present disclosure is a "carbon-free" energy that does not involve CO₂ emission during the entire process from production to use.

Furthermore, the hydrogen obtained using the alkane dehydrogenation catalyst can be used as a reducing agent for the reduction of a nitrogen oxide or the like. In addition, when a nitrogen oxide or the like is reduced using the alkane dehydrogenation catalyst, ammonia can be produced, and CO₂ is also not generated during the production stage of ammonia.

### Hydrogen production apparatus

The hydrogen production apparatus according to an embodiment of the present disclosure includes a means (or apparatus) for producing hydrogen using the method of producing hydrogen described above. Examples of the means or apparatus include a reaction vessel for reacting an alkane dehydrogenation catalyst and an alkane, a heating means (or a heating apparatus), a means for separating hydrogen and an alkane decomposition product from the product (or a separator), and a release means for releasing hydrogen that is separated.

Using the apparatus described above, it is possible to efficiently produce hydrogen using an alkane such as n-pentane or propane as a raw material with low energy while without generating CO₂. In addition, hydrogen can be released as needed. As such, the hydrogen production apparatus can be used as an apparatus for supplying hydrogen to a fuel cell that uses hydrogen as fuel, and the fuel cell can be used as a power source for, for example, a fuel cell vehicle.

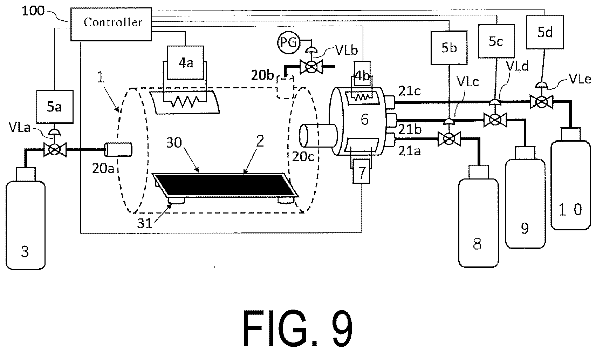

An example of the hydrogen production apparatus is illustrated in FIG. 9. The hydrogen production apparatus includes a reaction vessel 1 in which a base member 30 supporting an alkane dehydrogenation catalyst 2 is fixed by a holding member 31, an alkane storage tank 3 in which an alkane serving as a raw material is stored, a reaction vessel heating apparatus 4a, an alkane pressure controller 5a, a hydrogen release pressure controller 5b, an alkane/alkyne release pressure controller 5c, a lower alkane release pressure controller 5d, a gas separator 6, a gas separator heating apparatus 4b, a gas separator cooling apparatus 7, a hydrogen storage tank 8, an alkene/alkyne storage tank 9, and a lower alkane storage tank 10. Furthermore, the hydrogen production apparatus includes a controller 100.

The reaction vessel 1 is provided with an alkane supply opening 20a, an emergency release opening 20b, and a production gas release opening 20c.

The alkane supply opening 20a is connected to an alkane supply valve VLa via a first alkane supply pipe member. In addition, the alkane supply valve VLa is connected to the alkane storage tank 3 via a second alkane supply pipe member. The alkane pressure controller 5a, which operates according to control by the controller 100, controls the alkane supply valve VLa and adjusts the amount of alkane supplied from the alkane storage tank 3 to the reaction vessel 1.

The emergency release opening 20b is connected to an emergency release valve VLb via a first emergency release pipe member. Note that the emergency release valve VLb is closed during normal operation. When the measurement result of the pressure in the reaction vessel 1 by a pressure gauge PG exceeds a predetermined value, the emergency release valve VLb is brought into an open state. Gas passed through the emergency release valve VLb is then released to the outside via a second emergency release pipe member.

The production gas release opening 20c is connected to the gas separator 6 via a production gas pipe member.

The gas separator 6 is provided with an inlet opening for connecting with the production gas release opening 20c, a hydrogen release opening 21a, an alkene/alkyne release opening 21b, and a lower alkane release opening 21c.

The hydrogen release opening 21a is connected to a first hydrogen release valve VLc via a first hydrogen release pipe member. In addition, the first hydrogen release valve VLc is connected to the hydrogen storage tank 8 via a second hydrogen release pipe member. The hydrogen release pressure controller 5b, which operates according to control by the controller 100, adjusts the amount of hydrogen supplied from the gas separator 6 to the hydrogen storage tank 8 by controlling the release pressure of the produced hydrogen.

The alkene/alkyne release opening 21b is connected to a alkene/alkyne release valve VLd via a first alkene/alkyne release pipe member. In addition, the alkene/alkyne release valve VLd is connected to the alkane/alkyne storage tank 9 via a second alkene/alkyne release pipe member. The alkene/alkyne pressure controller 5c, which operates according to control by the controller 100, adjusts the amount of alkane and/or alkyne supplied from the gas separator 6 to the alkane/alkyne storage tank 9 by controlling the release pressure of the produced alkene and/or alkyne.

The lower alkane release opening 21c is connected to a lower alkane release valve VLe via a first lower alkane release pipe member. In addition, the lower alkane release valve VLe is connected to the lower alkane storage tank 10 via a second lower alkane release pipe member. The lower alkane pressure controller 5d, which operates according to control by the controller 100, adjusts the amount of lower alkane supplied from the gas separator 6 to the lower alkane storage tank 10 by controlling the release pressure of the produced lower alkane.

The reaction vessel 1 is sealed with the base member 30 housed therein. The alkane dehydrogenation catalyst 2, which has been adjusted into a powder, is fixed to a surface of the base member 30 or a mesh-like catalyst support structure using a support such as a metal with low activity, graphite, or alumina.

Note that a mass spectrometer may be provided to confirm that hydrogen is contained in the production gas. The mass spectrometer can be installed in, for example, a separate chamber that can be separated from the reaction vessel 1 with a gate valve. Moreover, production gas can be guided to the separate chamber via a heat resistant membrane or tube (for example, a palladium membrane or a palladium tube).

When production gas produced in the reaction vessel 1 is guided through the production gas release opening 20c into the gas separator 6, the gas is separated into hydrogen, a mixed gas of alkene and/or alkyne, and a lower alkane by a gas separation membrane. Each gas separated is released, with the hydrogen being released from the hydrogen release opening 21a, the mixed gas of the alkene and/or alkyne being released from the alkene/alkyne release opening 21b, and the lower alkane being released from the lower alkane release opening 21c. Here, the temperature inside the gas separator 6 is controlled by the gas separator heating apparatus 4b and the gas separator cooling apparatus 7 that are controlled through the controller 100.

As the gas separation membrane, a porous or non-porous polymer membrane such as polyimide, a porous or non-porous silica membrane, a porous or non-porous zeolite membrane, or a porous or non-porous carbon membrane can be used; one of the membranes can be used alone, or a combination of two or more thereof can be used.

The reaction vessel 1 can be replaced with a reaction vessel 11 having a mesh-like catalyst support structure for the purpose of increasing a contact area between an alkane serving as a raw material and the catalyst.

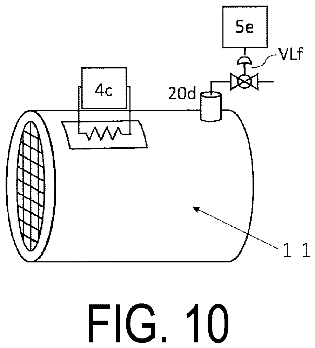

An example of the reaction vessel 11 having a mesh-like catalyst support structure is illustrated in FIG. 10. A heating apparatus 4c, a pressure controller 5e, and a release opening 20d are attached to the reaction vessel 11.

Similar to the reaction vessel 1, the reaction vessel 11 is connected to the alkane supply opening 20a and the production gas release opening 20c. The alkane supply opening 20a is further connected to the alkane supply valve VLa via the first alkane supply pipe member. The production gas release opening 20c is further connected to the gas separator 6 via the production gas pipe member.

According to the hydrogen production apparatus, by raising the temperature inside the reaction vessel 1 or the reaction vessel 11 to approximately from 300 to 750°C, it is possible to, using an alkane as a raw material, store hydrogen in the alkane dehydrogenation catalyst while producing an alkene and/or an alkene and a lower alkane at the same time. In addition, the stored hydrogen can be released from the alkane dehydrogenation catalyst by raising the temperature inside the reaction vessel 1 or the reaction vessel 11 to approximately from 650 to 1200°C. The reaction pressure at this time is, for example, approximately from 1 to 1500 kPa. The released hydrogen is released from the production gas release opening 20c, separated from the lower alkane, alkene, and alkyne by the gas separator 6, and stored in the hydrogen storage tank 8.

By utilizing a hydrogen production apparatus having the configuration described above, hydrogen can be produced without generating CO₂, and hydrogen can be safely stored and extracted as needed.

### Method of producing ammonia and apparatus for producing ammonia

The hydrogen obtained without generating CO₂ using the hydrogen production method according to an embodiment of the present disclosure (or hydrogen obtained without generating CO₂ using the hydrogen production apparatus according to an embodiment of the present disclosure) can be suitably used as, for example, a reducing agent. Furthermore, when the hydrogen is used as a reducing agent of a nitrogen oxide NOₓ (NO, NO₂, or the like), ammonia can be produced without generating CO₂.

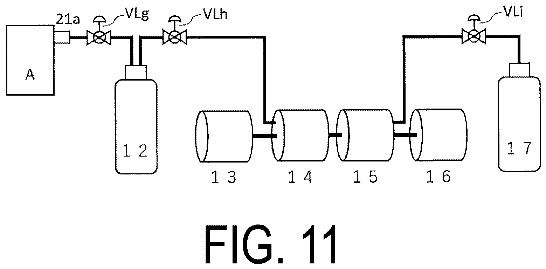

An ammonia production apparatus according to an embodiment of the present disclosure is provided with a means for producing ammonia using the method of producing hydrogen. An example of the ammonia production apparatus is illustrated in FIG. 11. The ammonia production apparatus includes a hydrogen production apparatus A having a hydrogen release opening 21a, a hydrogen supply valve VLg, a hydrogen buffer 12, a second hydrogen supply valve VLh, a NOₓ supply apparatus 13, a NOₓ reduction apparatus 14, an ammonia separator 15, a second exhaust purification apparatus 16, an ammonia supply valve VLi, and an ammonia storage tank 17.

The hydrogen production apparatus A is an apparatus that is the same as the hydrogen production apparatus except that the hydrogen release opening 21a is not included. The hydrogen release opening 21a is connected to a second hydrogen release valve VLg via a third hydrogen release pipe member, the third hydrogen release pipe member being connected to the hydrogen release opening 21a after the first hydrogen release pipe member is removed. Furthermore, the second hydrogen release valve VLg is connected to the hydrogen buffer 12 via a fourth hydrogen release pipe member.

Meanwhile, the NOₓ supply apparatus 13 is an apparatus that supplies a mixed gas of an inert gas (that is, a gas that is inert to the reaction with NOₓ or hydrogen, and examples thereof include nitrogen gas, helium gas, and argon gas) and NOₓ to the NOₓ reduction apparatus 14; for example, an apparatus capable of selectively extracting the mixed gas from the exhaust gas of a boiler or the exhaust gas of an internal combustion engine and supplying the mixed gas to the NOₓ reduction apparatus 14 can be used.

The hydrogen buffer 12 is connected to the second hydrogen supply valve VLh via a first hydrogen supply pipe member. Furthermore, the second hydrogen supply valve VLh is connected to the NOₓ reduction apparatus 14 via a second hydrogen supply pipe member.

The NOₓ reduction apparatus 14 may include a catalyst support base member supporting a NOₓ reduction catalyst that activates the reaction of NOₓ and hydrogen. The NOₓ reduction catalyst may be Cu-ZSM-5, or alumina, or a platinum group catalyst such as platinum. As a result of a reduction reaction using hydrogen supplied from the hydrogen production apparatus A as a reducing agent, a reaction gas containing ammonia is obtained.

The NOₓ reduction apparatus 14 is connected to the ammonia separator 15 via a reaction gas release member.

The ammonia separator 15 causes the ammonia contained in the reaction gas to be trapped in water or an appropriate adsorption material, and separates the nitrogen gas contained in the reaction gas. The nitrogen gas is sent to the exhaust purification apparatus 16 via an exhaust supply pipe member, and is released to the outside after a trace amount of unreacted NOₓ or the like is purified.

When water is used to trap ammonia, ammonia is stored in the ammonia storage tank while dissolved in water. Furthermore, ammonia can be extracted by evaporating water. When an adsorption material is used to trap ammonia, ammonia is extracted by, for example, raising the temperature of the adsorption material, and is stored in the ammonia storage tank.

By utilizing the ammonia production apparatus, it is possible to produce ammonia without generating CO₂ by supplying an alkane and an NOₓ as raw materials.

Each of the configurations, combinations thereof, and the like according to the present disclosure are an example, and various additions, omissions, substitutions, and changes may be made as appropriate without departing from the gist of the present disclosure. Further, the present disclosure is not limited by the embodiments and is limited only by the claims.

### Examples

Hereinafter, the present disclosure will be described more specifically with reference to examples, but the present disclosure is not limited by these examples.

### Example 1 Production of alkane dehydrogenation catalyst

### Preparation of raw material graphene

First, an explosive attached with an electric detonator was placed inside a pressure-resistant vessel (made of iron, volume: 15 m³) for detonation, and the vessel was sealed. As the explosive, 0.50 kg of a mixture of TNT and RDX (TNT/RDX (mass ratio) = 50/50) was used. Next, the electric detonator was triggered, and the explosive was detonated in the vessel. Subsequently, the vessel was allowed to stand at room temperature for 24 hours, and the temperatures of the vessel and the inside of the vessel were lowered. After the cooling, a crude graphene (containing graphene and impurities generated by the detonation method described above) deposited on the inner wall of the vessel was collected.

The obtained crude graphene was washed once with water and subjected to drying under reduced pressure. Thereafter, a precipitate obtained by heating and washing with 20% hydrochloric acid and centrifuging was subjected to drying under reduced pressure and further annealed at 800°C for 180 minutes, resulting in a purified graphene. The purified graphene was used as a raw material graphene.

The resulting purified graphene was dispersed in CS₂, resulting in a dispersion. Next, by a drop casting method, the obtained dispersion was used to form a film on a substrate having conductivity, resulting in a thin film graphene.

### Sputtering treatment

Next, argon gas was placed in a vacuum vessel (2 × 10⁻³ Pa), and irradiation with argon ions (0.4 µA) accelerated by an ion acceleration gun (ion acceleration voltage: 100 eV) was carried out for 30 minutes. This resulted in a catalyst (1) containing V/graphene having approximately one atomic vacancy structure per 1 nm² of the thin film graphene. Note that the amount of the atomic vacancy structures introduced was estimated from the total ion current amount with a probability of vacancy formation by ions being 100%.

### Quantification of hydrogen

The amount of hydrogen in the obtained catalyst (1), measured by the RBS/ERDA method under the following conditions, was 9 × 10¹⁵ atoms/cm².

### Measurement conditions

Incident ion: Helium ion (1.8 MeV)
Recoil ion: Hydrogen ion
Helium ion filter material: Aluminum
Incident beam angle: 75°
Recoil angle: 30°

### Example 2 Production of alkane dehydrogenation catalyst

A catalyst (2) containing V/graphene was obtained in the same manner as in Example 1 with the exceptions that the raw material graphene used was not the graphene obtained by a detonation method but instead a multilayer epitaxial graphene synthesized by heating a SiC substrate (trade name "SiC Single Crystal wafer", available from Nippon Steel & Sumikin Materials Co., Ltd.) at 2150°C and that the irradiation time of argon ions was changed to 5 minutes. The amount of hydrogen contained in the catalyst (2) was 1.2 × 10¹⁶ atoms/cm².

### Example 3 Production of alkane dehydrogenation catalyst

A catalyst (3) containing V_{N}/graphene and V/graphene was obtained using the same purified graphene as in Example 1 as the raw material graphene and in the same manner as in Example 1 with the exception that sputtering is performed by setting the position where an atomic vacancy is formed to the position of a carbon atom adjacent to a nitrogen atom in the purified graphene. The amount of hydrogen contained in the catalyst (3) was 9 × 10¹⁵ atoms/cm². In addition, the nitrogen content was 4 wt.% of the total amount of the catalyst (3).

### Example 4 Production of hydrogen

5 µg of the catalyst (2) obtained in Example 2, serving as a catalyst, and 8 g of butane were charged into a reaction vessel and reacted for 30 minutes at room temperature under normal pressure. After completion of the reaction, the catalyst containing V₁/graphene and V₁₁₁/graphene was retrieved, and the amount of hydrogen was measured by the RBS/ERDA method.

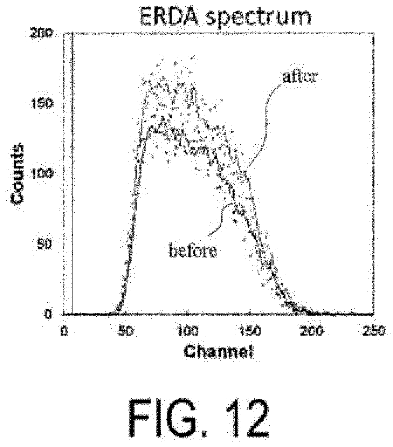

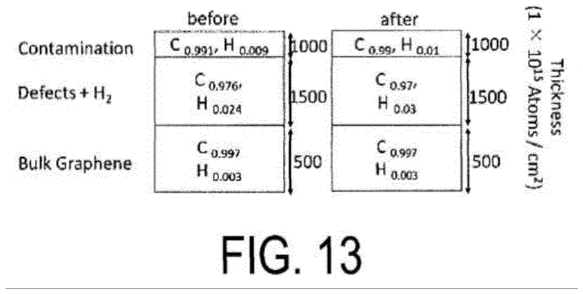

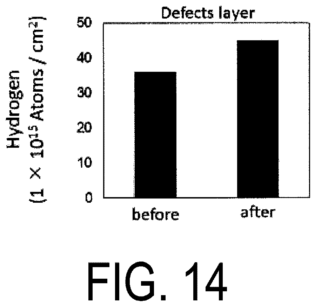

Then, the amount of hydrogen produced was calculated by subtracting the amount of hydrogen contained in the catalyst before the butane was reacted from the amount of hydrogen contained in the catalyst after the butane was reacted. The results are illustrated in FIGS. 12 to 14.

From FIGS. 12 to 14, it can be seen that hydrogen extracted from butane was stored in the atomic vacancy sites of the catalyst.

### Example 5 Production of hydrogen

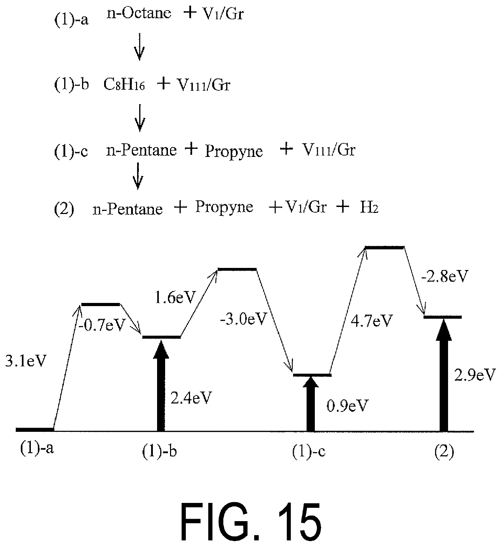

745 g of a catalyst (4) containing V₁/graphene obtained after the completion of the reaction of Example 4, serving as a catalyst, was reacted with 114 g of n-octane; the reaction path and the activation barrier for the above case were calculated by an electronic state calculation based on the Density Functional Theory. The results are illustrated in FIG. 15.

In addition, the activation barrier of a thermal decomposition reaction in which n-octane is decomposed into n-pentane and propyne does not fall below 5 eV. Therefore, heating at a high temperature of approximately from 700 to 1500°C would be necessary. However, from FIG. 15, it can be seen that by using the alkane dehydrogenation catalyst according to an embodiment of the present disclosure, the activation barrier was lowered to 3.1 eV, and the reaction proceeded at a mild temperature of approximately from 450 to 700°C.

### Example 6 Production of hydrogen

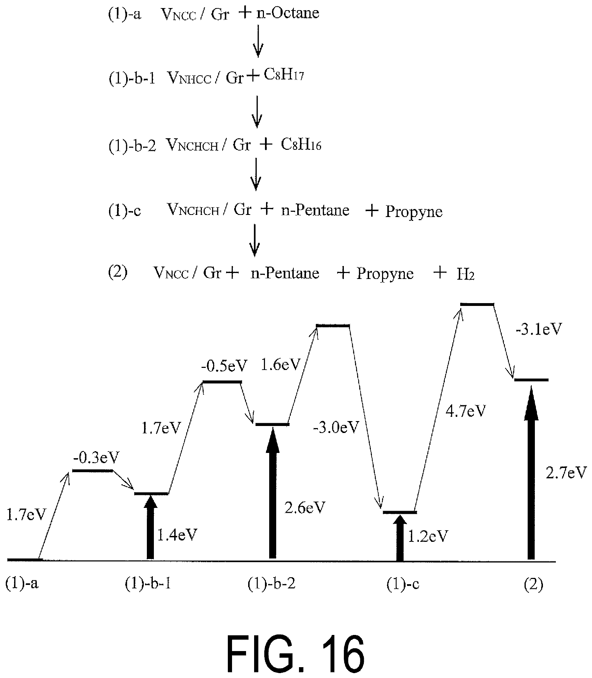

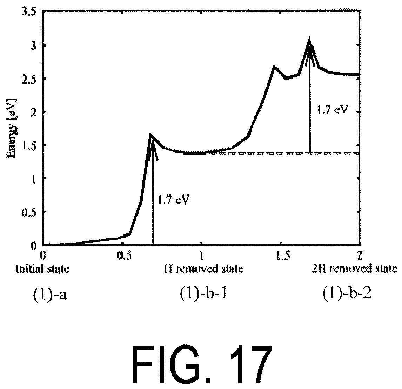

The same procedure as in Example 5 was performed except that the catalyst (3) obtained in Example 3 was used as a catalyst. The results are illustrated in FIGS. 16 and 17.

From FIGS. 16 and 17, it can be seen that in the thermal decomposition reaction in which n-octane is decomposed into n-pentane and propyne, the stage of obtaining C₈H₁₆ from n-octane was rendered into multiple stages; as such, the activation barrier at each stage became even smaller and the reaction proceeded at a milder temperature.

### Example 7 Production of hydrogen

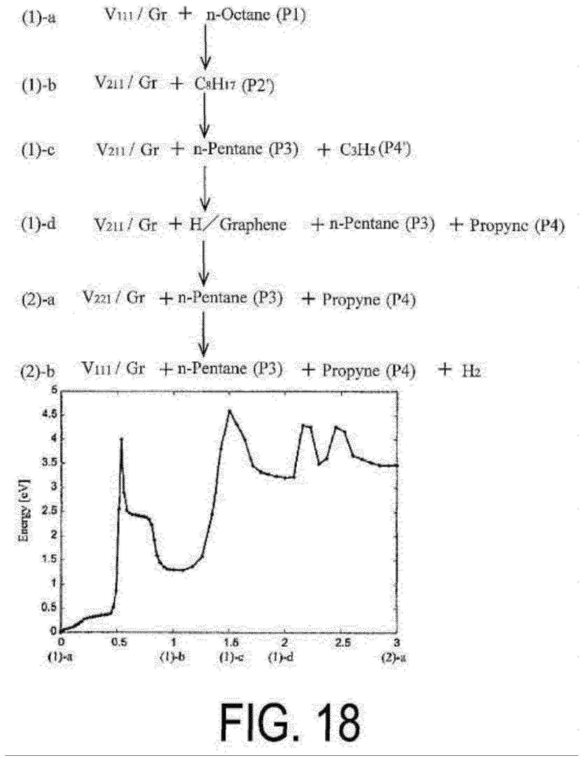

The same procedure as in Example 5 was performed except that a catalyst (5) containing V₁₁₁/graphene obtained after the completion of the reaction in Example 4 was used as a catalyst. The results are illustrated in FIG. 18.

From FIG. 18, it can be seen that in the thermal decomposition reaction in which n-octane is decomposed into n-pentane and propyne, hydrogen adsorption to graphene surface occurred at the stage of obtaining n-pentane and propyne from n-octane through C₈H₁₇; as such, the activation barrier of hydrogen diffusion and hydrogen desorption became even smaller, and the reaction proceeded under hydrogen partial pressure in a higher gas phase.

To summarize the above, configurations and variations according to an embodiment of the present disclosure will be described below.
[1] A graphene having at least one type of structure selected from: an atomic vacancy structure; a singly hydrogenated vacancy structure; a doubly hydrogenated vacancy structure; a triply hydrogenated vacancy structure; and a nitrogen-substituted vacancy structure.
[2] The graphene according to [1], wherein the graphene has from 2 to 200 of the at least one type of structure selected from: an atomic vacancy structure; a singly hydrogenated vacancy structure; a doubly hydrogenated vacancy structure; a triply hydrogenated vacancy structure; and a nitrogen-substituted vacancy structure, per 100 nm² of an atomic film of the graphene.
[3] The graphene according to [1] or [2], which is an alkane dehydrogenation catalyst.
[4] A use of the graphene according to [1] or [2] as an alkane dehydrogenation catalyst.
[5] A method of producing graphene, including colliding high-energy particles with a raw material graphene to obtain the graphene according to any one of [1] to [3].
[6] The method of producing graphene according to [5], wherein the raw material graphene is a graphene obtained by a detonation method.
[7] A method of producing hydrogen, including extracting hydrogen from an alkane using the graphene according to [1] or [2].
[8] The method of producing hydrogen according to [7], further including adsorbing-storing the hydrogen extracted from an alkane in an atomic vacancy site of the graphene.
[9] A hydrogen production apparatus, producing hydrogen using the method according to [7] or [8].
[10] An alkane dehydrogenation catalyst including a graphene, the graphene having at least one type of structure selected from: an atomic vacancy structure; a singly hydrogenated vacancy structure; a doubly hydrogenated vacancy structure; a triply hydrogenated vacancy structure; and a nitrogen-substituted vacancy structure.
[11] The alkane dehydrogenation catalyst according to [10], wherein the graphene has from 2 to 200 of the at least one type of structure selected from: an atomic vacancy structure; a singly hydrogenated vacancy structure; a doubly hydrogenated vacancy structure; a triply hydrogenated vacancy structure; and a nitrogen-substituted vacancy structure, per 100 nm² of an atomic film of the graphene.
[12] A method of producing an alkane dehydrogenation catalyst, including colliding high-energy particles with a raw material graphene to obtain the alkane dehydrogenation catalyst according to [10] or [11].
[13] The method of producing an alkane dehydrogenation catalyst according to [12], wherein the raw material graphene is a graphene obtained by a detonation method.
[14] A method of producing hydrogen, including extracting hydrogen from an alkane using the alkane dehydrogenation catalyst according to [10] or [11].
[15] The method of producing hydrogen according to [14], further including adsorbing-storing the hydrogen extracted from an alkane in an atomic vacancy site of the graphene.
[16] A method of producing ammonia, including producing hydrogen by the method according to any one of [7], [8], [14], and [15], and reducing a nitrogen oxide using the produced hydrogen to obtain ammonia.
[17] An ammonia production apparatus, producing ammonia using the method according to [16].

### Industrial Applicability

The alkane dehydrogenation catalyst according to an embodiment of the present disclosure enables extraction of hydrogen from an alkane without emitting CO₂ and without requiring significant energy. The hydrogen obtained is extremely useful as a renewable energy; even when the hydrogen is burned and used as thermal energy, CO₂ is not emitted.

### Reference Signs List

1 Reaction vessel
2 Alkane dehydrogenation catalyst
3 Alkane storage tank
4a Reaction vessel heating apparatus
4b Gas separator heating apparatus
4c Heating apparatus
5a Alkane pressure controller
5b Hydrogen release pressure controller
5c Alkane/alkyne release pressure controller
5d Lower alkane release pressure controller
5e Pressure controller
6 Gas separator
7 Gas separator cooling apparatus
8 Hydrogen storage tank
9 Alkene/alkyne storage tank
10 Lower alkane storage tank
11 Reaction vessel
12 Hydrogen buffer
13 NOₓ supply apparatus
14 NOₓ reduction apparatus
15 Ammonia separator
16 Second exhaust purification apparatus
17 Ammonia storage tank
20a Alkane supply opening
20b Emergency release opening
20c Production gas release opening
20d Release opening
21a Hydrogen release opening
21b Alkene/alkyne release opening
21c Lower alkane release opening
30 Base member
31 Holding member
100 Controller
VLa Alkane supply valve
VLb Emergency release valve
VLd Alkene/alkyne release valve
VLe Lower alkane release valve
PG Pressure gauge
A Hydrogen production apparatus
VLg Hydrogen supply valve
VLh Second hydrogen supply valve
VLi Ammonia supply valve

## Claims

1. An alkane dehydrogenation catalyst comprising a graphene, the graphene having at least one type of structure selected from: an atomic vacancy structure; a singly hydrogenated vacancy structure; a doubly hydrogenated vacancy structure a triply hydrogenated vacancy structure; and a nitrogen-substituted vacancy structure.

2. The alkane dehydrogenation catalyst according to claim 1, wherein the graphene has from 2 to 200 of the at least one type of structure selected from: an atomic vacancy structure; a singly hydrogenated vacancy structure; a doubly hydrogenated vacancy structure; a triply hydrogenated vacancy structure; and a nitrogen-substituted vacancy structure, per 100 nm² of an atomic film of the graphene.

3. A method of producing an alkane dehydrogenation catalyst, comprising colliding high-energy particles with a raw material graphene to obtain the alkane dehydrogenation catalyst according to claim 1 or 2.

4. The method of producing an alkane dehydrogenation catalyst according to claim 3, wherein the raw material graphene is a graphene obtained by a detonation method.

5. A method of producing hydrogen, comprising extracting hydrogen from an alkane using the alkane dehydrogenation catalyst according to claim 1 or 2.

6. The method of producing hydrogen according to claim 5, further comprising adsorbing-storing the hydrogen extracted from an alkane in an atomic vacancy site of the graphene.

7. A hydrogen production apparatus producing hydrogen using the method according to claim 5 or 6.

8. A method of producing ammonia, comprising producing hydrogen by the method according to claim 5 or 6, and reducing a nitrogen oxide using the produced hydrogen to obtain ammonia.

9. An ammonia production apparatus, producing ammonia using the method according to claim 8.
